# EUROPEAN PATENT SPECIFICATION

(11) **EP 2 350 340 B1**
(45) Date of publication and mention of the grant of the patent: **27.03.2019**
(21) Application number: 09815395.0
(22) Date of filing: 22.09.2009
(51) Int. Cl.: C23C 16/00, A61M 5/31, B05D 7/22, C23C 16/04, C23C 16/40, B05D 1/00

(54) **SYSTEMS FOR COATING THE INTERIOR OF A CONTAINER**
SYSTEME ZUR BESCHICHTUNG DER INNENSEITE EINES BEHÄLTERS
SYSTÈMES PERMETTANT DE REVÊTIR L'INTÉRIEUR D'UN CONTENANT

(30) Priority: 22.09.2008 US 192782 P
(43) Date of publication of application: 03.08.2011
(60) Divisional application: 19153689.5
(73) Proprietor: Becton, Dickinson and Company, Franklin Lakes, NJ 07417-1880 (US)
(72) Inventor: RODRIGUEZ SAN JUAN, Nestor, Hamburg, New Jersey 07419 (US); SRIDHARAN, Srinivasan, Mendham, New Jersey 07945 (US)
(74) Representative: dompatent von Kreisler Selting Werner - Partnerschaft von Patent- und Rechtsanwälten mbB
(86) International application number: PCT/US2009/057886
(87) International publication number: WO 2010/034004

(56) References cited:
- JP-A- 2008 127 054
- US-A- 4 435 445
- US-A1- 2003 138 645
- US-B1- 6 331 174
- US-B2- 6 916 502

## Description

### BACKGROUND OF THE INVENTION

### Field of the Invention

This invention relates to systems, apparatus and methods for photolysis and/or thermal chemical vapor deposition (CVD), such as pyrolyzing hot filament CVD (HFCVD), to provide barrier and/or lubricant coating(s) on the interior surface of a container, such as a syringe, tube or medical collection device.

### Description of Related Art

Traditionally, containers for chemically sensitive materials have been made from inorganic materials such as glass. Glass containers offer the advantage that they are substantially impenetrable by atmospheric gases and thus provide a product with a long shelf life. However, glass containers can be fragile and expensive to manufacture.

More recently, lighter and less expensive containers made of polymeric materials are being used in applications in which traditional glass containers were used. These polymeric containers are less susceptible to breakage, less expensive to manufacture, lighter and less expensive to ship than glass containers. However, polymeric containers can be permeable to gases, permitting atmospheric gases to pass through the polymeric container to the packaged product and also permitting gases in the packaged product to escape through the polymeric container, both of which undesirably degrade the quality and shelf life of the packaged product.

One approach to decrease the gas permeability of polymeric containers is to form a multilayered polymeric container which includes at least one low gas permeable polymeric layer along with at least one other polymeric layer. However, such an approach is relatively complicated and costly.

Whether the container is formed from glass or polymeric material, reactivity of the interior surface of the container with the contents of the container, such as biological materials and/or drugs, can be problematic. Trace components of the glass or polymeric material may migrate into the container contents, and/or components of the container contents may migrate or react with the interior surface of the container.

To decrease migration of container components and/or contents, and to reduce the gas permeability of polymeric containers, a barrier coating may be deposited on the interior of the container, i.e., a coating having a substantial resistance to the permeation of gaseous or volatile material through the polymeric container or resistance to migration between the container interior surface and the container contents. However, it can be difficult to obtain a uniform barrier coating, particularly on the interior of containers of small volume and complex interior design, for example syringe barrels.

Also, certain devices, such as syringe barrels, require slow and controlled initiation and maintenance of sliding movement of one surface over another surface. It is well known that two stationary surfaces having a sliding relationship often exhibit sufficient resistance to initiation of movement that gradually increased force applied to one of the surfaces does not cause movement until a threshold force is reached, at which point a sudden sliding or shearing separation of the surfaces takes place. This sudden separation of stationary surfaces into a sliding relationship is herein referred to as "breakout" or "breakloose".

"Breakout force" refers to the force required to overcome static friction between surfaces of a syringe assembly that has been previously moved in a sliding relationship, but has been stationary ("parked" or not moved) for a short period of time (for example, milliseconds to hours). A less well known but important frictional force is "breakloose force", which refers to the force required to overcome static friction between surfaces of a syringe assembly that have not been previously moved in a sliding relationship or have been stationary for longer periods of time, often with chemical or material bonding or deformation of the surfaces due to age, sterilization, temperature cycling, or other processing.

Breakout and breakloose forces are particularly troublesome in liquid dispensing devices, such as syringes, used to deliver small, accurately measured quantities of a liquid by smooth incremental line to line advancement of one surface over a second surface. The problem is also encountered in devices using stopcocks, such as burets, pipets, addition funnels, and the like where careful dropwise control of flow is desired.

The problems of excessive breakout and breakloose forces are related to friction. Friction is generally defined as the resisting force that arises when a surface of one substance slides, or tends to slide, over an adjoining surface of itself or another substance. Between surfaces of solids in contact, there may be two kinds of friction: (1) the resistance opposing the force required to start to move one surface over another, conventionally known as static friction, and (2) the resistance opposing the force required to move one surface over another at a variable, fixed, or predetermined speed, conventionally known as kinetic friction.

The force required to overcome static friction and induce breakout or breakloose is referred to as the "breakout force" or "breakloose force", respectively, and the force required to maintain steady slide of one surface over another after breakout or breakloose is referred to as the "sustaining force". Two main factors, sticktion and inertia, contribute to static friction and thus to the breakout or breakloose force. The term "stick" or "sticktion" as used herein denotes the tendency of two surfaces in stationary contact to develop a degree of adherence to each other. The term "inertia" is conventionally defined as the indisposition to motion which must be overcome to set a mass in motion. In the context of the present invention, inertia is understood to denote that component of the breakout or breakloose force which does not involve adherence.

Breakout or breakloose forces, in particular the degree of stick, vary according to the composition of the surfaces. In general, materials having elasticity show greater stick than non-elastic materials. The length of time that surfaces have been in stationary contact with each other also influences breakout and/or breakloose forces. In the syringe art, the term "parking" denotes storage time, shelf time, or the interval between filling and discharge. Parking time generally increases breakout or breakloose force, particularly if the syringe has been refrigerated or heated during parking.

A conventional approach to overcoming breakout or breakloose has been application of a lubricant to a surface interface. Common lubricants used are hydrocarbon oils, such as mineral oils, peanut oil, vegetable oils, and the like. Such products have the disadvantage of being soluble in a variety of fluids, such as vehicles commonly used to dispense medicaments. In addition, these lubricants are subject to air oxidation resulting in viscosity changes and objectionable color development. Further, they are particularly likely to migrate from the surface to surface interface. Such lubricant migration is generally thought to be responsible for the increase in breakout or breakloose force with time in parking.

Silicone oils are commonly used as lubricants, and have the advantage of not being subject to oxidation, however migration and stick do occur, and high breakout and/or breakloose forces can be a problem. Polytetrafluoroethylene surfaces provide some reduction in breakout and/or breakloose forces, but this material is very expensive, and the approach has not been totally effective.

Thus, there is a need for a better system to overcome high breakout and breakloose forces whereby smooth transition of two surfaces from stationary contact into sliding contact can be achieved. Also, there is a need for a simple, inexpensive and reliable process for depositing a barrier coating on the interior of a container to prevent leaching of materials from the container surface into the container contents and/or from the container contents into the container surface, and to prevent gas and/or water permeability in containers, such as syringes, tubes and medical collection devices

JP 2008 127054 A discloses a system for coating recyclable plastic bottles comprising a vacuum chamber with a container to be coated, a fluorocarbon monomer gas supply, a pyrolyzing surface in the form of a hot filament, a temperature controller with cooling lines on the inside of vacuum chamber and an outlet duct.

### SUMMARY OF THE INVENTION

The present invention relates to a system according to claim 1. In some non-limiting embodiments, the present disclosure provides a system for coating at least a portion of an interior wall surface of a container, comprising: (a) a container comprising an open end, a second end opposite the open end, and a wall extending therebetween, the wall having an exterior wall surface and an interior wall surface, the container having a chamber within an area defined by the interior wall surface between the open end and the second end of the container; (b) a monomer gas supply source for supplying at least one monomer gas; (c) a gas inlet duct positioned at the open end of the container and having a portion extending into a portion of the chamber for supplying at least one monomer gas received from the monomer gas supply source into the chamber; (d) a pyrolyzing surface for pyrolyzing at least a portion of the monomer gas supplied to the chamber of the container to form a reactive gas comprising at least one reactive moiety from the monomer gas; (e) a temperature controller for maintaining the interior wall surface of the container at a temperature which is less than the temperature of the pyrolyzing surface to facilitate deposition and polymerization of the reactive moiety on at least a portion of the interior wall surface of the container; and (f) an outlet duct positioned at the open end of the container or the second end of the container for removing excess reactive gas from the chamber.

In some non-limiting embodiments, the present disclosure provides an apparatus for coating at least a portion of an interior wall surface of a container, the container comprising an open end, a second end opposite the open end, and a wall extending therebetween, the wall having an exterior wall surface and an interior wall surface, the container having a chamber within an area defined by the interior wall surface between the open end and the second end of the container, the apparatus comprising: (a) a monomer gas supply source for supplying at least one monomer gas; (b) a gas inlet duct for being positioned at the open end of the container, the gas inlet duct having a portion for being extended into a portion of the chamber for supplying at least one monomer gas received from the monomer gas supply source into the chamber; (c) a pyrolyzing surface for pyrolyzing at least a portion of the monomer gas supplied to the chamber of the container to form a reactive gas comprising at least one reactive moiety from the monomer gas; (d) a temperature controller for maintaining the interior wall surface of the container at a temperature which is less than the temperature of the pyrolyzing surface to facilitate deposition and polymerization of the reactive moiety on at least a portion of the interior wall surface of the container; and (e) an outlet duct for being positioned at the open end of the container or the second end of the container for removing excess reactive gas from the chamber.

In some non-limiting embodiments, the present disclosure a provides a system for coating at least a portion of an interior wall surface of a container, comprising: (a) a container comprising an open end, a second end opposite the open end, and a wall extending therebetween, the wall having an exterior wall surface and an interior wall surface, the container having a chamber within an area defined by the interior wall surface between the open end and the second end of the container; (b) a monomer gas supply source for supplying at least one monomer gas; (c) a gas inlet duct positioned at the open end of the container and having a portion extending into a portion of the chamber for supplying at least one monomer gas received from the monomer gas supply source into the chamber; (d) a photolysis energy source for providing photons to at least a portion of the monomer gas supplied to the chamber of the container to form a reactive gas comprising at least one reactive moiety from the monomer gas; and (e) an outlet duct positioned at the open end of the container or the second end of the container for removing excess reactive gas from the chamber.

In some non-limiting embodiments, the present disclosure provides an apparatus for coating at least a portion of an interior wall surface of a container, the container comprising an open end, a second end opposite the open end, and a wall extending therebetween, the wall having an exterior wall surface and an interior wall surface, the container having a chamber within an area defined by the interior wall surface between the open end and the second end of the container, the apparatus comprising: (a) a monomer gas supply source for supplying at least one monomer gas; (b) a gas inlet duct for being positioned at the open end of the container, the gas inlet duct having a portion for being extended into a portion of the chamber for supplying at least one monomer gas received from the monomer gas supply source into the chamber; (c) a photolysis energy source for providing photons to at least a portion of the monomer gas supplied to the chamber of the container to form a reactive gas comprising at least one reactive moiety from the monomer gas; and (d) an outlet duct for being positioned at the open end of the container or the second end of the container for removing excess reactive gas from the chamber.

In some non-limiting embodiments, the present disclosure provides a method for applying a film coating to an interior wall surface of a container, comprising: (a) providing a monomer gas supply source for supplying at least one monomer gas to a gas inlet duct; (b) positioning a container comprising an open end, a second end opposite the open end, and a wall extending therebetween, the wall having an exterior wall surface and an interior wall surface, the container having a chamber within an area defined by the interior wall surface between the open end and the second end of the container, such that the open end is connected to the monomer gas supply source by the gas inlet duct; (c) positioning the gas inlet duct at the open end of the container such that a portion of the gas inlet duct extends into a portion of the chamber for supplying at least one monomer gas received from the monomer gas supply source into the chamber; (d) pyrolyzing at least a portion of the monomer gas supplied to the chamber of the container to form a reactive gas comprising at least one reactive moiety; (e) maintaining the interior wall surface of the container at a temperature which is less than the temperature of the pyrolyzing gas to facilitate deposition and polymerization of the reactive moiety on at least a portion of the interior wall surface of the container; and (f) removing excess reactive gas from the chamber through an outlet duct positioned at the open end of the container or the second end of the container.

In some non-limiting embodiments, the present disclosure provides a method for applying a film coating to an interior wall surface of a container, comprising: (a) providing a monomer gas supply source for supplying at least one monomer gas to a gas inlet duct; (b) positioning a container comprising an open end, a second end opposite the open end, and a wall extending therebetween, the wall having an exterior wall surface and an interior wall surface, the container having a chamber within an area defined by the interior wall surface between the open end and the second end of the container, such that the open end is connected to the monomer gas supply source by the gas inlet duct; (c) positioning the gas inlet duct at the open end of the container such that a portion of the gas inlet duct extends into a portion of the chamber for supplying at least one monomer gas received from the monomer gas supply source into the chamber; (d) photolyzing at least a portion of the monomer gas supplied to the chamber of the container to form a reactive gas comprising at least one reactive moiety; and (e) removing excess reactive gas from the chamber through an outlet duct positioned at the open end of the container or the second end of the container.

In some non-limiting embodiments, the present disclosure provides a medical article comprising a chamber having an interior wall surface and at least one coating layer thereon, wherein the coating layer has a first region deposited from a first reactive gas having a chemical functionality that is reactive with a first surface functionality present in a first domain of the interior wall surface prior to coating and a second region deposited from a second reactive gas having a chemical functionality that is reactive with a second surface functionality present in a second domain of the interior wall surface prior to coating.

In some non-limiting embodiments, the present disclosure provides a method for applying a film coating to an interior wall surface of a container, comprising: (a) providing a container comprising an interior wall surface, the interior wall surface comprising a first domain having a first chemical functionality and a second domain having a second, different chemical functionality; (b) exposing the interior wall surface to a first reactive gas having a functionality reactive with the first chemical functionality of the first domain and a second reactive gas having a functionality reactive with the second chemical functionality of the second domain, either sequentially or simultaneously; and (c) exposing the coated interior wall surface to an energy source to facilitate formation of the coating layer.

### BRIEF DESCRIPTION OF THE DRAWINGS

The present invention will best be understood from the following description of specific embodiments when read in connection with the accompanying drawings:
Fig. 1 is a schematic diagram of a system and apparatus for coating the interior of a container using a chemical vapor deposition process;
Fig. 2 is a schematic diagram of an alternative embodiment of system and apparatus for coating the interior of a container using a chemical vapor deposition process;
Fig. 3 is schematic diagram of a portion of the system and apparatus of Fig. 1 for coating the interior of a single container using a chemical vapor deposition process;
Fig. 4 is a schematic diagram of an alternative embodiment of a system and apparatus of Fig. 1 for coating the interior of a single container using a chemical vapor deposition process;
Fig. 5 is a schematic diagram of an alternative embodiment of a system and apparatus of Fig. 1 for coating the interior of a single container using a chemical vapor deposition process;
Fig. 6 is a side elevational view of a portion of a sidewall of a container having a first coating and a second coating thereon;
Fig. 7 is a side elevational view of a portion of a sidewall of a container having regions of different chemical functionalities and a coating layer having corresponding regions of different chemical functionalities;
Fig. 8 is a bottom elevational view of a plate surrounding a cross section of a gas inlet duct for promoting radial flow of gas from the gas inlet duct;
Fig. 9 is a schematic diagram of a system and apparatus for coating the interior of a container using a photolysis deposition process; and
Fig. 10 is a schematic diagram of a system and apparatus for coating the interior of a container using a photolysis and a chemical vapor deposition process.

### DETAILED DESCRIPTION

Other than in the operating examples, or where otherwise indicated, all numbers expressing quantities of ingredients, reaction conditions, and so forth used in the specification and claims are to be understood as being modified in all instances by the term "about". Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical values, however, inherently contain certain errors necessarily resulting from the standard deviation found in their respective testing measurements. Furthermore, when numerical ranges of varying scope are set forth herein, it is contemplated that any combination of these values inclusive of the recited values may be used.

Also, it should be understood that any numerical range recited herein is intended to include all sub-ranges subsumed therein. For example, a range of "1 to 10" is intended to include all sub-ranges between and including the recited minimum value of 1 and the recited maximum value of 10, that is, having a minimum value equal to or greater than 1 and a maximum value of equal to or less than 10.

A notable feature of the present invention is that the container acts as its own individual vacuum chamber, wherein photolysis and/or pyrolyzing CVD induced or enhanced reactions take place with the resulting modification of, or deposition on, the interior wall surface of the container. The systems, apparatus and methods of the present disclosure do not require a vacuum chamber. A vacuum chamber such as is used in most deposition processes, requires significant process space and control.

The present invention increases the efficiency of manufacturing by allowing in-line processing on each individual container, as opposed to the usual method of batch processing of many containers. Large batch processing vacuum chambers require longer pump-down times both due to the increased chamber volume and de-gassing the chamber. Therefore, with the present invention, loading and unloading of containers into and out of a batch processing chamber is eliminated.

Another useful feature of the present invention is that the barrier film coating on the interior wall surface of the container is substantially protected from physical damage. When the barrier film coating is on the outside of the container, which is the usual case, it is subject to abrasive damage due to handling during manufacture, shipping, or by the end user. Therefore, a barrier film coating on the interior wall surface of the container improves the effectiveness of the shelf-life of the container because damage to the barrier film coating is substantially reduced.

Another advantage of the present invention is that the barrier film coating on the interior wall surface is of substantially higher quality than a barrier film coating on the outside wall of the container. This is due to the fact that the interior wall surface of the container is less likely to be subject to contact contamination, such as oils, greases and dust, as is the outside of the container during manufacture. Such contaminations on the walls of the container could cause coating non-uniformity, defects, and poor adhesion. No cleaning of accumulated coatings or particulates is required for the inside of the container, since each container to be coated is a new treatment "chamber".

Another advantage of the present invention is that the means for imparting energy inside the container may be altered, moved and/or rotated inside the container in various locations and positions, to substantially assure uniformity of the barrier film coating. Therefore, "shadowing" during plasma process is not an issue as it is in plasma processing on the outside wall of the container.

Other drawbacks of batch systems wherein processing is on the outside wall of the container, include container "fit" over electrodes and variations in container dimensions, such as "bow". These issues are not a problem in the present invention. Furthermore, failure of a large batch processing unit would result in a large loss in productivity, whereas failure of an in-line unit would be a minor loss of capacity if many lines were available (since each line costs less). Due to the simplicity of the in-line process, ruggedness and repairability are improved over the alternative batch process. It is believed that containers could be in-line processed by this invention without ever stopping movement along the line

A further advantage of the present invention is that since the means for imparting energy inside the container is internal to the container, such apparatus is easily and inexpensively altered. This allows tailoring a production line to specific requirements, by easily altering such apparatus as gas inlet duct dimensions, in conjunction with altering the reactant gases. This would allow a single line to process different container configurations with only minor changes in the production line.

Plastic tubes coated on the interior wall surface with the barrier film coating can maintain substantially better vacuum retention, draw volume and thermomechanical integrity retention than plastic tubes comprised of polymer compositions and blends thereof with a barrier film coating on the external wall surface of the tube. In addition, the resistance of the tube to impact is substantially much better than that of glass. Most notable is the clarity of the barrier film coating and its durability to substantially withstand resistance to impact and abrasion, such as during shipping and handling for syringes, use in automated machinery such as centrifuges for testing tubes and/or exposure to certain levels of radiation in the sterilization process.

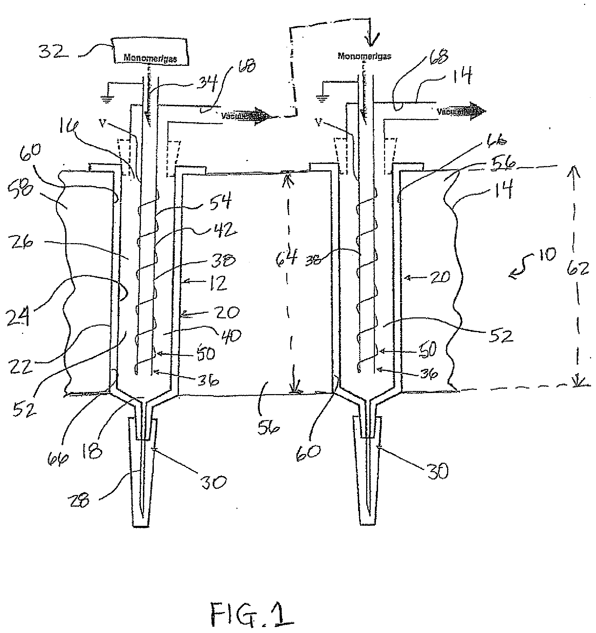

Referring now to Fig. 1, wherein like numeral indicate like elements throughout, in some non-limiting embodiments, the present disclosure provides a system, generally indicated as 10, for coating at least a portion of an interior wall surface of a container. The system 10 comprises at least one container 12 and an apparatus 14 for coating at least a portion of an interior wall surface of the container. The container 10 comprises an open end 16, a second end 18 opposite the open end 16, and a wall 20 extending therebetween, the wall 20 having an exterior wall surface 22 and an interior wall surface 24. The container 12 has a chamber 26 within an area defined by the interior wall surface 24 between the open end 16 and the second end 18 of the container 12.

The container 12 can be of any shape, for example generally cylindrical, tubular, or cup. The container has a first, at least partially open end 16 and a second end 18 generally opposite the first end 16. The second end 18 can be open or closed.

In some non-limiting embodiments, the container is selected from the group consisting of syringes, drug cartridges, needleless injectors, liquid dispensing devices, liquid metering devices, sample collection tubes, catheters, vials and tubing. For example, as shown in Fig. 1, the container 12 can comprise a syringe barrel. The syringe barrel container 12 can have an open end 16 and a second, generally opposite end 18 which can be closed, for example by having an attachable needle 28 or cannula attached thereto, and optionally a shield or tip cap 30 covering at least a portion of the exterior of the attachable needle 28 or cannula. For example, the syringe barrel, needle and tip cap assembly can be those used in the STERIFILL SCF™ plastic prefillable syringe systems or HYPAK SCF™ glass prefillable syringe systems which are available from Becton Dickinson Company of Franklin Lakes, New Jersey.

The container can be formed from glass, metal, ceramic, plastic, rubber, or combinations thereof. In some non-limiting embodiments, the chamber is prepared from one or more olefinic polymers, such as polyethylene, polypropylene, poly(1-butene), poly(2-methyl-1-pentene), and/or cyclic polyolefin. For example, the polyolefin can be a homopolymer or a copolymer of an aliphatic monoolefin, the aliphatic monoolefin preferably having about 2 to 6 carbon atoms, such as polypropylene. In some non-limiting embodiments, the polyolefin can be basically linear, but optionally may contain side chains such as are found, for instance, in conventional, low density polyethylene. In some non-limiting embodiments, the polyolefin is at least 50% isotactic. In other embodiments, the polyolefin is at least about 90% isotactic in structure. In some non-limiting embodiments, syndiotactic polymers can be used. In some non-limiting embodiments, cyclic polyolefins can be used. Non-limiting examples of suitable cyclic polyolefins include norbornene polymers such as are disclosed in U.S. Patents Nos. 6,525,144, 6,511,756, 5,599,882, and 5,034,482 (each of Nippon Zeon), 7,037,993, 6,995,226, 6,908,970, 6,653,424 and 6,486,264 (each of Zeon Corp.), 7,026,401 and 6,951,898 (Ticona), 6,063,886 (Mitsui Chemicals), 5,866,662, 5,856,414, 5,623,039 and 5,610,253 (Hoechst), 5,854,349 and 5,650,471 (Mitsui Petrochemical and Hoechst) and as described in "Polycyclic olefins", Process Economics Program (July 1998) SRI Consulting, each of the foregoing references being incorporated by reference herein. Non-limiting examples of suitable cyclic polyolefins include Apel™ cyclic polyolefins available from Mitsui Petrochemical, Topas™ cyclic polyolefins available from Ticona Engineering Polymers, Zeonor™ or Zeonex™ cyclic polyolefins available from Zeon Corporation, and cyclic polyolefins available from Promerus LLC.

The polyolefin can contain a small amount, generally from about 0.1 to 10 percent, of an additional polymer incorporated into the composition by copolymerization with the appropriate monomer. Such copolymers may be added to the composition to enhance other characteristics of the final composition, and may be, for example, polyacrylate, polystyrene, SEBS, and the like.

In some non-limiting embodiments, the chamber may be constructed of a polyolefin composition which includes a radiation stabilizing additive to impart radiation stability to the container, such as a mobilizing additive which contributes to the radiation stability of the container, such as for example those disclosed in U.S. Patent Nos. 4,959,402 and 4,994,552, assigned to Becton, Dickinson and Company and both of which are incorporated herein by reference.

In some non-limiting embodiments, the container of the present disclosure is a blood collection device. The blood collection device can be either an evacuated blood collection tube or a non-evacuated blood collection tube. The blood collection tube can be made of polyethyleneterephthalate, polypropylene, polyethylene naphthalate or copolymers thereof.

The dimensions, e.g., inner and outer diameter, length, wall thickness, etc. of the container 12 can be of any size desired. The dimensions of the gas inlet duct and flow rate of reactive gas into the chamber of the container can be adjusted to accommodate the dimensions and desired coating thickness for any chamber. For example, for a one ml volume syringe barrel, the inner diameter of the barrel is about 0.25 inches (6.35 mm) and the length is about 2.0 inches (50.8 mm). For a plastic Sterifill 20 ml volume syringe barrel, the inner diameter of the barrel is about 0.75 inches (19.05 mm) and the length is about 3.75 inches (95.3 mm). Generally, the inner diameter can range from about 0.25 inches (6.35 mm) to about 10 inches (254 mm), or about 0.25 inches (6.35 mm) to about 5 inches (127 mm), or any value therebetween. Thus, the outside diameter of the gas inlet duct must be less than the inner diameter of the container to permit flow of the reactive gas therebetween and to permit the hot filament (if present) to be positioned about the duct.

Since the delivery of the activated gas is achieved in close proximity to the surface to be coated, no flow driving regime is required and very low gas flows can be used. The gas flow rate will be determined by the desired deposition rate, the vacuum capacity, the effective pumping cross section allowed by the device/duct, and the line time-process allowances. Typical flow rates per nozzle generally can range from about 1 to about 1000 ml/min.

As discussed above, the apparatus 14 provides photolysing and/or thermal- or pyrolyzing-CVD induced or enhanced reactions which take place within the chamber of each individual container to deposit and polymerize reactive moieties on the interior wall surface of the container.

The apparatus 14 comprises at least one monomer gas supply source 32 for supplying at least one monomer gas 34, through a gas inlet duct 36. The monomer gas can be selected to provide the desired coating on the interior of the container. In some non-limiting embodiments, the monomer gas comprises at least one monomer selected from the group consisting of organosilicon monomers, halocarbon monomers, azurine monomers, thiirane monomers, unsaturated olefinic monomers and mixtures thereof. Non-limiting examples of suitable monomer gases are disclosed in U.S. Patents Nos. 6,153,269, 6,156,435, and 6,887,578, each of which is incorporated by reference herein in its entirety.

The term "fluorocarbon" as used herein means a halocarbon compound in which fluorine replaces some or all hydrogen atoms. The term "organosilicon" as used herein means a compound containing at least one Si--C bond.

Non-limiting examples of suitable organosilicon monomers include those selected from the group consisting of hexamethylcyclotrisiloxane, octamethylcyclotetrasiloxane, 1,3,5-trivinyl-1,3,5-trimethylcyclotrisiloxane, 1,3,5,7-tetravinyl-1,3,5,7-tetramethylcyclotrisiloxane, 3-(N-allylamino)propyltrimethoxysilane, allyldichlorosilane, allyldimethoxysilane, allyldimethylsilane, allyltrichlorosilane, allyltrimethoxysilane, allyltrimethylsilane, bis(dimethylamino)vinylmethylsilane, para-(t-butyldimethylsiloxy)styrene, decamethylcyclopentasiloxane, diethylsilane, dimethylethoxysilane, dimethylsilane, divinyldimethylsilane, divinyltetramethyldisilane, 1,3-divinyltetramethyldisiloxane, ethyltrimethoxysilane, hexamethyldisiloxane, 1,1,3,3,5,5-hexamethyltrisiloxane, hexavinyldisiloxane, methyltriethoxysilane, methyltrimethoxysilane, methylsilane, tetraethoxysilane, tetraethylcyclotetrasiloxane, tetraethylsilane, tetramethoxysilane, 1,1,3,3-tetramethyldisiloxane, tetramethylsilane, tetravinylsilane, trimethylsilane, vinyldimethylsilane, vinylmethylbis(trimethylsiloxy)-silane 3-vinylheptamethyltrisiloxane, vinylmethyldiethoxysilane, vinyloxytrimethylsilane, vinylpentamethyldisiloxane, vinyltetramethyldisiloxane, vinyltrimethoxysilane, vinyltrimethylsilane, and mixtures thereof.

Non-limiting examples of suitable halocarbon monomers include those selected from the group consisting of hexafluoropropylene oxide, tetrafluoroethylene, hexafluorocyclopropane, octafluorocyclobutane, perfluorooctanesulfonyl fluoride, octafluoropropane, trifluoromethane, difluoromethane, difluorodichloromethane, difluorodibromomethane, difluorobromomethane, difluorochloromethane, trifluorochloromethane, tetrafluorocyclopropane, tetrachlorodifluorocyclopropane, trichlorotrifluoroethane, dichlorotetrafluorocyclopropane and mixtures thereof.

Non-limiting examples of suitable unsaturated olefin monomers include those selected from the group consisting of dicyclopentadiene (DCP), dipentadiene, norbornene, cyclopentadiene, methyltetracyclododecene (MTD), tetracyclododecene, and mixtures thereof.

In some non-limiting embodiments, the deposition processes provided by the present disclosure enable tailoring of the chemical composition of deposited films to produce fluorocarbon polymer thin films having stoichiometry and materials properties similar to that of bulk PTFE. One useful monomer is hexafluoropropylene oxide (C₃F₆O or HFPO). HFPO is characterized by a highly-strained epoxide ring that enables easy ring-opening reactions with nucleophiles. It has been found that films deposited using HFPO under HFCVD conditions result in polymer films having a high CF₂ fraction and little or no oxygen incorporation.

In some non-limiting embodiments, the processes of the present disclosure contemplates use of any feed gas that provides a monomer which can be photolyzed or pyrolyzed to provide difluorocarbene species (CF₂) for producing a fluorocarbon polymer film having a high fraction of CF₂ groups and a low degree of polymer crosslinking. For example, the HFPO monomer described above is understood to decompose under pyrolysis to form a fluorinated ketone and the desired difluorocarbene. The fluorinated ketone is relatively stable, compared with the difluorocarbene. This is understood to lead to a high CF₂ content in a film as polymerization occurs at the film deposition surface. Oxygen present in the monomer is tied up in the relatively unreactive ketone decomposition byproduct, whereby little oxygen is incorporated into the film.

Considering the selection of gas monomer in general, the ratio of CFₓ/F in the gas can effect the competing deposition and etching reactions that occur during a deposition process; a higher ratio can correspond to enhancement of deposition and suppression of etching reactions. This ratio can be increased by including in a feed gas composition a fluorine scavenger, e.g., hydrogen, a hydrocarbon, or an unsaturated compound or monomer. In general, the addition of hydrogen or C₂F₄ to a fluorocarbon feed gas can result in decreasing atomic F concentration relative to CFₓ concentration. This decreased atomic F concentration can result in an increased deposition rate. Additionally, the inclusion of hydrogen in the feed gas can alter the gap-filling capabilities of the deposited film due to its reduction in ion bombardment. Furthermore, hydrogen can be included in a feed gas to provide an in situ mechanism for passivating dangling bonds on the surface of a structure being processed. For example, hydrogen can passivate amorphous silicon dangling bonds. In some non-limiting embodiments, use of less reactive, but not interfering, radicals than the difluorocarbene or the use of a on-off deposition scheme where the input of the gases is alternated can be used. Since the present methods use relative lower flow and higher efficiency that HFCVD, the on-off scheme can be controlled more accurately.

The selection of feed gas constituents should also preferably take into consideration any trace impurities that could be incorporated into a film deposited from the feed gas. For example, HFPO as a feed gas monomer can result in incorporation of trace amount of oxygen in a deposited film. Thus, if trace oxygen is not acceptable for a deposited film, a feed gas monomer other than HFPO is preferable. Other process parameters should likewise preferably be considered in selecting a feed gas monomer, as will be recognized by those skilled in the art.

In some non-limiting embodiments, the monomer gas can comprise azurines or thiiranes, for example 3-methyl-2*H*-azirine-2-methyl, 3-amino--2*H*-azirine-2-methyl, 2-hydroxy-2*H*-azirine, 3-phenyl-2*H*-azirine, 2,3-Diarylthiirene 1-oxide, 2,3-di-*t*-butylthiirrene 1-oxide, and 2,3-Dimethylthiirene.

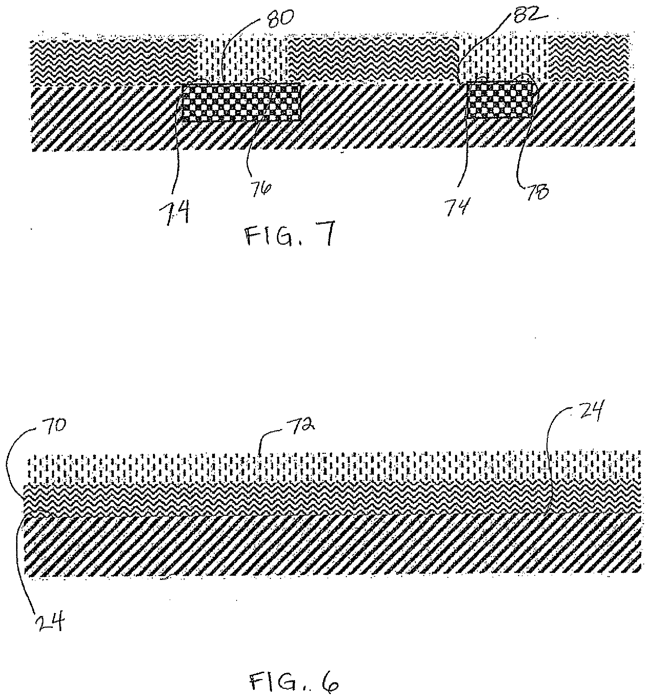

Referring now to Fig. 6, chemically different monomer gases can be input into the chamber 26 sequentially to deposit multiple layers upon the substrate. As shown in Fig. 6, a first layer 70 can be deposited from a first reactive gas and a second layer 72 can be deposited thereon from a second, chemically different monomer gas. Alternatively, if a blend of reactive gases is used, the volume ratio of the blend can be adjusted during deposition to form multiple layers or a gradient layer. Non-limiting examples of multilayer deposition materials deposition of a mixture of polyethylene and siloxane as a first layer and PTFE as a second layer, or PTFE as a first layer and poly acrylic acid as a second layer.

Referring now to Fig. 1, the apparatus 14 comprises a gas inlet duct 36 or nozzle positioned at the open end 16 of the container 12 and having a portion 38 extending into a portion 40 of the chamber 26 for supplying at least one monomer gas 34 received from the monomer gas supply source 32 into the chamber 26. The shape of the gas inlet duct 36 can be any shape desired, such as a generally cylindrical tube or conduit. The sidewall(s) 42 of the gas inlet duct 36 can be generally smooth or have protuberances to induce turbulent flow, as desired. In some non-limiting embodiments, gas inlet duct 36 has a plurality of apertures 44 in the sidewall 42 of the duct 36 or tube. In some non-limiting embodiments, the gas inlet duct is prepared from a porous material.

The gas inlet duct 36 can be formed from any metal, ceramic or high temperature resistant material plastic, such as stainless steel, zirconia, silicate ceramic, polysulfones polymer (such as UDEL™ polysulfones available from Solvay Polymers), polyether ether ketone (PEEK) or polyether imides. In some non-limiting embodiments, the gas inlet duct 36 can be formed from a material that functions as a catalyst to facilitate the radical polymerization and/or modification of the difluorocarbene from diradical singlet to triplet. Non-limiting examples of suitable functional materials include transition metals, such as chromium, copper and palladium. These metals can be obtained in cast type (solid) or sintered (porous material thermally bonded, for example using a cementing material like cobalt in the process or compacted to certain density). Sintered materials such as nichrome alloys, cuprothal alloys and constatan alloys can be used. Doped porous ceramic materials could also be used as catalysts. The duct 36 can be heated indirectly by an appropriate heating element or convection system or directly by passing an electric current therethrough, as discussed below. Induction type of indirect heating by an r.f. signal is possible including infrared or microwave range signals with the appropriate frequency and amplitude.

In some non-limiting embodiments, the apparatus 14 can further comprise a transition metal catalytic surface (not shown) positioned within the chamber 26, in addition to any transition metal surface that may be incorporated into the gas inlet duct 36. The transition metal catalytic surface can be an insert positioned at the lower end of the gas inlet duct 36 or along a side of the gas inlet duct 36, as desired. The transition metal catalytic surface can facilitate formation of the reactive moieties in the reactive gas.

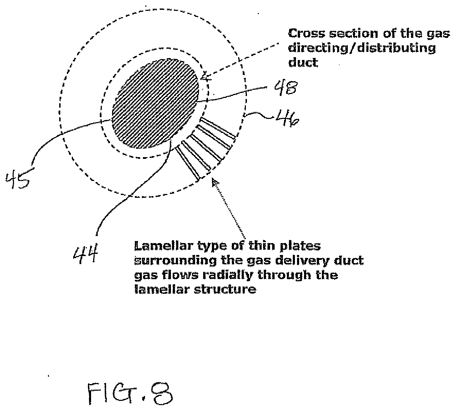

In some non-limiting embodiments, the gas inlet duct 36 further comprises at least one plate 46 (shown in Fig. 8) extending radially from an exterior sidewall 48 of the tube or duct 36 proximate an aperture 44 in the sidewall 48 of the tube 36. The plate 46 can be configured to surround the outer periphery of the duct 36. The plate 46 can include a plurality of radial fins or apertures therethrough for conducting a portion of the gas radially outwardly from the duct 36. The plate 46 can be formed from any of the materials discussed above as suitable for forming the duct, and can be formed from the same material as the duct, if desired.

The system and apparatus will now be discussed with reference to using pyrolysis for generating the reactive gas. Other systems and apparatus using photolysis for generating the reactive gas, and systems and apparatus for using both photolysis and pyrolysis for generating the reactive gas will be discussed in detail below.

Referring now to Fig. 1, the apparatus 14 comprises a pyrolyzing surface 50 for pyrolyzing at least a portion of the monomer gas 34 supplied to the chamber 26 of the container 12 to form a reactive gas 52 comprising at least one reactive moiety. The reactive moieties formed by pyrolyzation of the monomer gas discussed above are well known to those skilled in the art. For example, many of the fluorocarbon monomers discussed above can produce the difluorocarbene radical upon pyrolysis as discussed in detail above. In some non-limiting embodiments, the pyrolyzing surface 50 can be present in proximity with the gas inlet duct 36, as shown in Fig. 1. Alternatively or additionally, the pyrolyzing surface 50 can be present at any suitable position between the gas supply source 32 and the chamber 26, whereby the gas inlet duct 36 delivers the reactive gas 52 comprising at least one reactive moiety into the chamber 26. This pyrolysis technique has the advantage of eliminating a pyrolysis surface in the chamber that itself is coated with the reactive gas species produced by the pyrolysis.

The temperature of the pyrolyzing surface 50 should be sufficient to pyrolyze or combust at least a portion of the monomer gas 34 and form one or more reactive moieties. For example, the temperature of the pyrolyzing surface 50 can be about 300°K to about 773°K, or greater than about 500°K. One skilled in the art would understand that the monomer gas 34 need not directly contact the pyrolyzing surface 50, but can be at least partially pyrolyzed when in proximity to the heat generated by the pyrolyzing surface 50. Also, one skilled in the art would understand that, depending upon the monomer gas(es) selected, the pyrolysis temperature and duration of exposure may vary.

The term "chemical vapor deposition" as used herein means a process which transforms gaseous molecules or radicals into solid material in the form of thin film or powder on the surface of a substrate. In the thermal or hot filament chemical vapor deposition (thermal-CVD) process, substantially no ion bombardment occurs, because no substantial electric field is generated in the deposition chamber to attract the charged ions to the film as it is deposited. Notably, and in contrast to films deposited by PECVD, films deposited via hot-filament CVD (HFCVD) have well-defined compositions. For example, PECVD-deposited fluorocarbon films comprise a variety of CF groups (e.g., CF₃, tertiary C, and C--F, in addition to CF₂), while HFCVD-deposited fluorocarbon films consist almost entirely of CF₂, along with a small amount of CF₃ moieties. Further, the initiating and terminating groups in HFCVD are well-defined; whereas the precursors in PECVD processes undergo much greater fragmentation (these films have Si--F bonds, for instance, that result from total fragmentation of the fluorocarbon precursors). A consequence of the nature of the HFCVD process is that only the most thermally stable groups (e.g., CF₂ and siloxane rings) appear in the film, resulting in more thermally stable films. Photo-initiated CVD (ph-CVD), or photolysis, has the specificity of HFCVD with the advantage of minimal or no collateral thermal heat being added to the system allowing deposition under cooler conditions.

One of the most important specific chemical differences between hot-filament CVD and plasma-enhanced CVD is the occurrence of ion-bombardment and ultraviolet-irradiation in the latter technique. Due to this difference, HFCVD films do not contain defects seen in PECVD films. For example, HFCVD films do not have dangling bonds, which are always produced in PECVD processes. Dangling bonds are unpaired electrons left behind in the film. If such bonds are present, the film will undergo reactions with components of the ambient atmosphere (such as water, for instance, resulting in a large number of hydroxyl groups). Therefore, PECVD films are more susceptible to atmospheric ageing, and degradation of their optical, electrical and chemical properties. Moreover, films produced by HFCVD processes are less dense than those produced by plasma-enhanced CVD processes. Due to the differences between the nucleation and growth mechanisms the two processes, it is possible to make porous films using HFCVD, but not using PECVD.

The use of an initiator in HFCVD allows films to be deposited at significantly higher rates and provides greater control over chemical composition and morphology. This was demonstrated by Pryce Lewis et al. for fluorocarbon films deposited from hexafluoropropylene oxide (HFPO) using perfluorooctane sulfonyl fluoride (PFOSF) as an initiator. Pryce Lewis, H. G.; Caulfield, J. A.; Gleason, K. K. Langmuir 2001, 17, 7652. In the mechanism proposed for film growth, the generation of free radicals from the pyrolysis of PFOSF is the initiation step. The fluorocarbon radical subsequently combines with the propagating species, difluorocarbene (CF₂), which is generated by the pyrolysis of HFPO. The use of PFOSF resulted in higher deposition rates, more efficient utilization of HFPO, and endcapping by CF₃ groups.

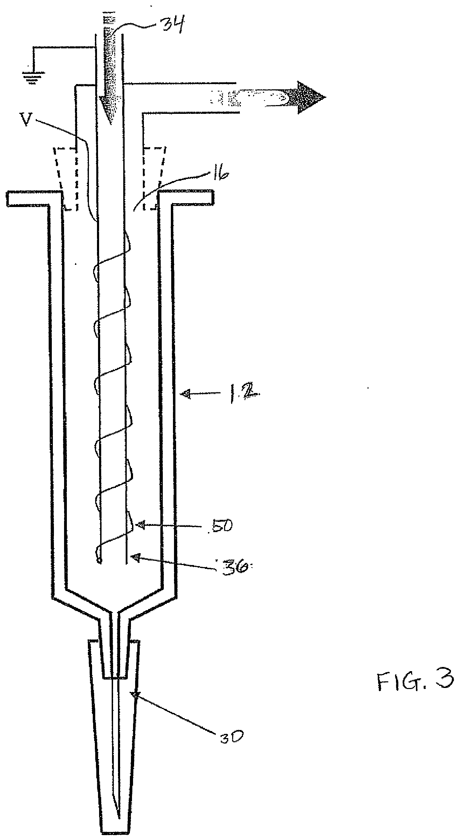

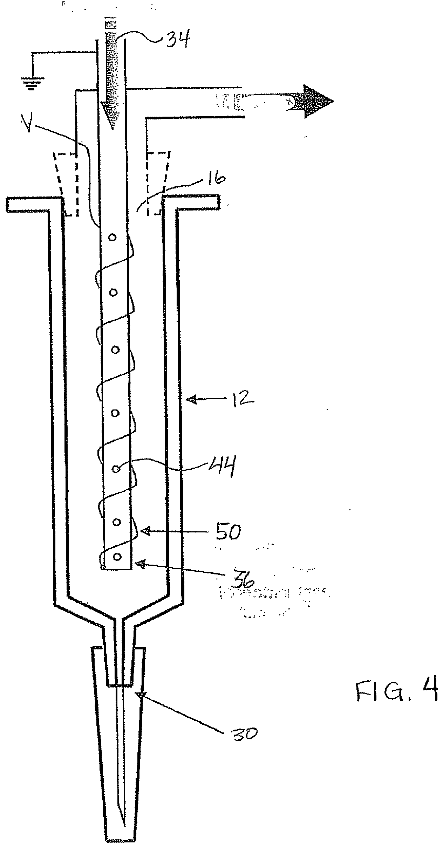

In some non-limiting embodiments, the pyrolyzing surface 50 can be a hot-filament, as shown in Figs. 1, 3 and 4. The hot-filament or other heated surface is preferably provided in a position relative to the input monomer gas flow such that the input monomer gas flows in the vicinity of the heated structure; whereby the gas is pyrolyzed to produce reactive deposition species. For example, as shown in Figs. 1, 3 and 4, a hot-filament 50 can be positioned or wound about an exterior surface 54 of the gas inlet duct 36, such that gas injected to the chamber 26 through the gas inlet duct 36 passes over the hot filament 50. The hot filament can be heated by, e.g., resistive heating. In this case, a dc voltage source (not shown) is provided to apply the heating voltage to the filament, consisting of, e.g., a Ni/Cr wire. The hot filament wire can have a diameter of about 0.3 to about 0.5 mm, for example, and a length to provide the appropriate ohmic resistance to adjust the temperature of the process.

Among the different CVD techniques available, hot-filament CVD (HFCVD, also known as pyrolytic or hot-wire CVD) is unique in several respects. In HFCVD, a precursor gas is thermally decomposed by a resistively heated filament. The resulting pyrolysis products adsorb onto a substrate maintained at around room temperature and react to form a film. HFCVD does not require the generation of plasma, thereby avoiding defects in the growing film produced by UV irradiation and ion bombardment. In addition, films produced by HFCVD have a better-defined chemical structure because there are fewer reaction pathways than in the less selective plasma-enhanced CVD method. HFCVD provides films with a substantially lower density of dangling bonds, i.e., unpaired electrons. Further, HFCVD has been shown to produce films that have a low degree of crosslinking. HFCVD has been used to deposit fluorocarbon films that are spectroscopically similar to poly(tetrafluoroethylene) (PTFE) as well as organosilicon films that consist of linear and cyclic siloxane repeat units. Limb, S. J., Lau, K. K. S., Edell, D. J., Gleason, E. F., Gleason, K. K. Plasmas and Polymers 1999, 4, 21.

Thermal excitation mechanisms other than a hot-filament are equally suitable for the thermal-CVD process. Indeed, it is preferable that the selected thermal mechanism, together with the gas delivery system, provide both uniform gas input and uniform pyrolysis of the gas. Hot windows, electrodes, or other surfaces, as well as heated walls 42 of the gas inlet duct 36, can alternatively be employed in pyrolysis configurations aimed at producing uniform gas pyrolysis. In some non-limiting embodiments, the pyrolyzing surface 50 is an integral part of the gas inlet duct. For example, the gas inlet duct 36 can be made from a metallic material and a portion thereof can be heated to a pyrolyzing temperature by convection or directly by passing an electric current therethrough, or induction type of indirect heating by an r.f. signal is possible including infrared or microwave range signals with the appropriate frequency and amplitude. Other direct heating techniques, e.g., laser heating techniques, can also be employed, as can be employed in general a wide range of other pyrolysis mechanisms.

As discussed above, the apparatus 14 comprises a temperature controller 56 for maintaining the interior wall surface 24 of the container 12 at a temperature which is less than the temperature of the pyrolyzing surface 50 or reactive gas 52 to facilitate deposition and polymerization of the reactive moiety on at least a portion of the interior wall surface 24 of the container 12. The temperature of the interior wall surface 24 of the container 12 is maintained at a temperature lower than the pyrolysis temperature of the pyrolyzing surface 50 or reactive gas 52. Specifically, the temperature of the interior wall surface 24 of the container 12 is preferably maintained low enough to favor polymerization under the partial pressure of a given reactive species employed in the deposition process. It is also preferable that the partial pressure of the reactive species be kept to a low level that prevents homogeneous gas-phase reactions, which could cause particle production in the gaseous environment rather than on the object surface to be coated.

In some non-limiting embodiments, the temperature of the interior wall surface 24 of the container 12 depends of the specific material being coated and the cross section for establishing covalent bonding between the radical species and the surface, e.g., for CCP resin the temperature must be lower than 140°C. The temperature of the interior wall surface 24 is less than the temperature of the pyrolyzing surface 50 or reactive gas 52, for example at least about 20°C or more less, and in some embodiments is held at a temperature of between about -40°C and about +200°C (233°K to 473°K) during the deposition; or about 20°C to about 50°C (293°K to 323°K). The temperature that is maintained during film deposition can be an important factor for determining the deposition rate, the stability of the radical species and the ultimate thermal stability of a film produced by the deposition process. Films deposited at relatively higher structural temperatures may in some applications be relatively more resistant to heating. The deposition time will depend on the flow rate, activation efficiency and targeted thickness of the coating. Typical deposition times can range from seconds to hours. Very fast deposition times are desirable to implement the on-off scheme deposition scheme mentioned above. The thickness of the film generally can range from about 1 nm to about 100 microns, for example.

As shown in Fig. 1, in some non-limiting embodiments, the temperature controller 56 is a thick metal plate 58 having at least one aperture 60 therethrough for receiving the container 12 therein such that the exterior wall surface 22 of the container 14 is proximate to or in facing engagement with the aperture 60 of the plate 58. The plate can be formed from any metal material, for example stainless steel or copper. Copper has the advantage of having high heat conductance and is therefore capable of cooling the container quickly and efficiently. The thickness 62 of the metal plate can be commensurate with the length 64 of the container 12, or less than the length 64 of the container 12. The internal surface 66 of each aperture 60 in the plate 58 generally conforms to the exterior shape of the respective container 12 positioned therein. The internal surface 66 of each aperture 60 can be the same or different, depending upon the exterior shape of the container 12 to be positioned therein. Also, the internal surface 66 of each aperture 60 is preferably proximate the exterior wall surface 22 of the container 14 or in facing engagement therewith to promote cooling or heat transfer as necessary to maintain the interior wall surface 24 of the container 12 at the desired temperature for the desired duration.

In other non-limiting embodiments, the temperature controller can be a thermal jacket positioned about the exterior wall surface 22 of each container 14. The thermal jacket can be formed from a metal material, such as copper. Since the apparatus can be used in an on-line process and the dimensions of the container will have dimensional variance, it is foreseen that some type of elastic interfacial layer could be used on the cooling element such as a high heat conductance polymer, polymer-filler like silicone rubber or polytetrafluorethylene-graphite filled polymer. Cooling coils, or other appropriate cooling mechanisms, can be employed to maintain interior wall surface 24 of the container 12 at the desired temperature for the desired duration.

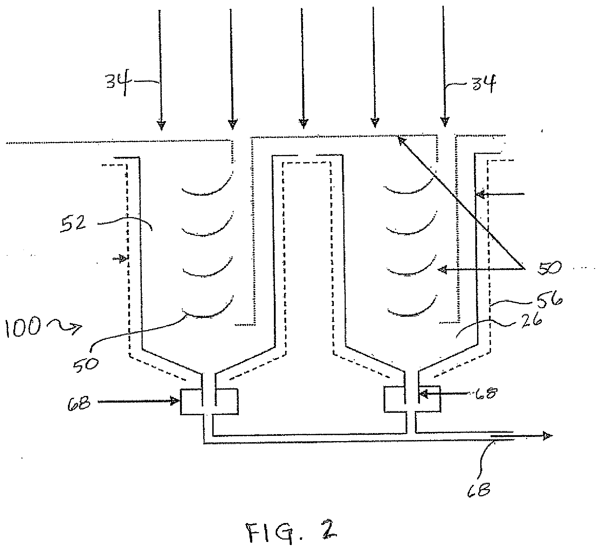

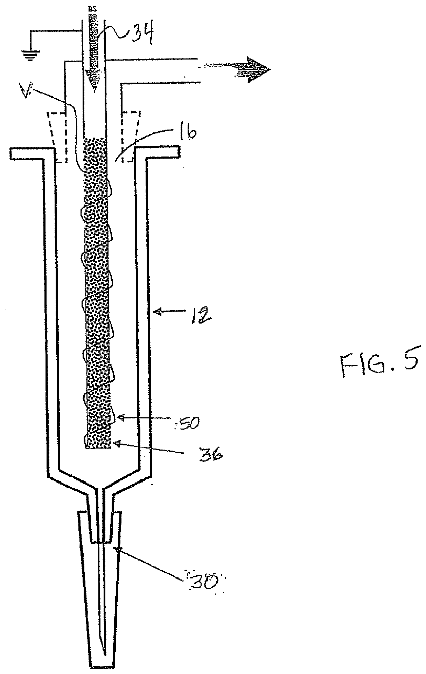

As shown in Figs. 1 and 3-5, the apparatus 14 comprises at least one outlet duct 68 positioned at the open end 16 of the container 12 or the second end 18 of the container (shown in Fig. 2) for removing excess reactive gas 52 as well as the byproducts of the pyrolysis/photolysis or activation reactions from the chamber 26. The removal of the excess reactive gas 52 and other pyrolysis products can be conducted at atmospheric pressure or under vacuum. The pressure will be determined by the ratio of the mean free path of the reactive species to the distance activation surface-to-substrate. The evacuation can be accomplished in two regimes, diffusive and molecular flow, and typical vacuum pressures can range from about 1.0 Pa to about 500 Pa. An additional enhancement of the apparatus would be the use of a cold trap (cryopump) close to the outlet duct 68 to increase pumping speed during the deposition cycle and follow by a desorbing step during the transition of the duct assembly head to the next rack. The outlet duct 68 can be connected to an evacuation system, or to a gas inlet duct inserted into an adjacent container, i.e., connected in series.

In some non-limiting embodiments, the system 10 further comprises at least one treatment selected from the group consisting of surface treatment (oxidative, noble gas or other), heat treatment, and irradiation with an isotope, electron beam, or ultraviolet radiation. This additional treatment can be carried out prior to, simultaneously with, or after the pyrolysis treatment. This treatment can promote adhesion (covalent or non-covalent bonding) or surface property modifications. Suitable apparatus can be used in conjunction with the existing apparatus 14 so that the containers do not need to be moved prior to additional treatment, or the containers may be additionally treated at a second location using additional equipment.

The plasma treatment may be carried out in any common vacuum or atmospheric plasma generation equipment. Any suitable ionizing plasma may be used, as, for example, a plasma generated by a glow discharge or a corona discharge. The plasma may be generated from a variety of gases or mixtures thereof. Gases frequently used include air, hydrogen, helium, ammonia, nitrogen, oxygen, neon, argon, krypton, and xenon. Any gas pressure may be used, for example, atmospheric pressure or 5 mm of Hg or below, such as about 0.1 to about 1.0 mm of Hg. In some embodiments such as atmospheric oxidative methods, the ionizing plasma can be introduced directly from a small port at the opening in the chamber. The interior wall surface 24 of the container 12 can be treated directly similarly to current corona or plasma treatment methods. In other embodiments, such as vacuum based equipment, the plasma can be excited around the coated chamber and allowed to diffuse into the chamber features. Alternatively, the plasma may be excited within the interior of the open chamber by properly controlling electrode position. After oxidative treatment, the treated chamber can be subjected to heat treatment or irradiation with an isotope (such as gamma radiation), electron beam, or ultraviolet radiation. Alternatively, the treated chamber can be heat treated via oven or radio frequency (RF). In the case of oven crosslinking, temperatures can range from about 120° to about 140° C and residence time in the oven is generally about 30 to about 40 seconds, depending on the precise formulation. If RF techniques are used, the coil should conduct enough heat to obtain a substrate surface temperature of about 150° to about 200°C. At these temperatures, only about 2 to about 4 seconds are required for cure.

In some embodiments, the coating is at least partially crosslinked by irradiation with an isotope, electron beam, or ultraviolet radiation. This technique has the advantage of sterilizing as well, which is useful in medical applications. Radiation sterilization in the form of ionizing radiation commonly is used in hospitals for medical devices such as catheters, surgical items, and critical care tools. Gamma irradiation exerts a microbicidal effect by oxidizing biological tissue, and thus provides a simple, rapid and efficacious method of sterilization. Gamma rays are used either from a cobalt-60 (⁶⁰Co) isotope source or from a machine-generated accelerated electron source. Sufficient exposures are achieved when the materials to be sterilized are moved around an exposed ⁶⁰Co source for a defined period of time. The most commonly used dose for sterilizing medical articles is about 5 to about 100 kGy, for example, 5-50 kGy.

In some non-limiting embodiments, the present disclosure provides a method for applying a film coating to an interior wall surface of a container, comprising: (a) providing a monomer gas supply source for supplying at least one monomer gas to a gas inlet duct; (b) positioning a container comprising an open end, a second end opposite the open end, and a wall extending therebetween, the wall having an exterior wall surface and an interior wall surface, the container having a chamber within an area defined by the interior wall surface between the open end and the second end of the container, such that the open end is connected to the monomer gas supply source by the gas inlet duct; (c) positioning the gas inlet duct at the open end of the container such that a portion of the gas inlet duct extends into a portion of the chamber for supplying at least one monomer gas received from the monomer gas supply source into the chamber; (d) pyrolyzing at least a portion of the monomer gas supplied to the chamber of the container to form a reactive gas comprising at least one reactive moiety; (e) maintaining the interior wall surface of the container at a temperature which is less than the temperature of the pyrolyzing gas to facilitate deposition and polymerization of the reactive moiety on at least a portion of the interior wall surface of the container; and (f) removing excess reactive gas from the chamber under vacuum through a vacuum outlet duct positioned at the open end of the container or the second end of the container.

In some non-limiting embodiments, the method further comprises treating at least a portion of the interior wall surface of the container to at least one treatment selected from the group consisting of oxidative treatment, heat treatment, and irradiation with an isotope, electron beam, or ultraviolet radiation prior to, simultaneously with, or after the pyrolysis treatment, as discussed in detail above.

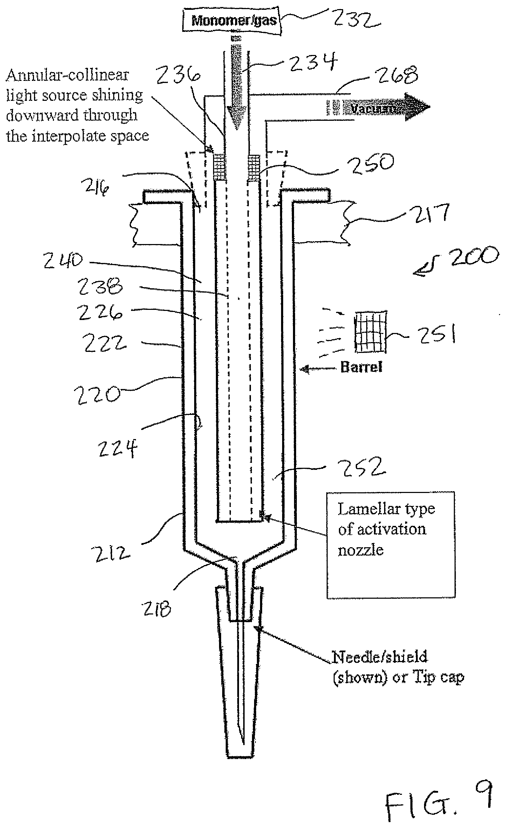

Referring now to Fig. 9, a system using photolysis will now be discussed. In some non-limiting embodiments, the present disclosure provides a system, indicated generally as 200, for coating at least a portion of an interior wall surface 224 of a container 212, comprising: (a) a container 212 comprising an open end 216 supported by a support 217, a second end 218 opposite the open end 216, and a wall 220 extending therebetween, the wall 220 having an exterior wall surface 222 and an interior wall surface 224, the container 212 having a chamber 226 within an area defined by the interior wall surface 224 between the open end 216 and the second end 218 of the container 212; (b) a monomer gas supply source 232 for supplying at least one monomer gas 234; (c) a gas inlet duct 236 positioned at the open end 216 of the container 212 and having a portion 238 extending into a portion 240 of the chamber 226 for supplying at least one monomer gas 234 received from the monomer gas supply source 232 into the chamber 226; (d) a photolysis energy source 250 for providing photons to at least a portion of the monomer gas 234 supplied to the chamber 226 of the container 212 to form a reactive gas 252 comprising at least one reactive moiety from the monomer gas 234; and (e) an outlet duct 268 positioned at the open end 216 of the container 212 or the second end 218 of the container 212 for removing excess reactive gas from the chamber 226. The system optionally can comprise a temperature controller 256 for maintaining the interior wall surface 224 of the container 312 at a temperature which is less than the temperature of the photolysis energy source 250 to facilitate deposition and polymerization of the reactive moiety on at least a portion of the interior wall surface 224 of the container 212.

The components of the system other than the photolysis energy source are the same as those described in detail above with respect to the pyrolysis system and apparatus.

In some non-limiting embodiments, the photolysis energy source has a predetermined wavelength (or range of wavelengths). In some non-limiting embodiments, the photolysis energy source is ultraviolet radiation having a predetermined wavelength within the ultraviolet range. In some non-limiting embodiments, the photolysis energy source is gamma radiation having a predetermined wavelength within the gamma range.

In some non-limiting embodiments, the photolysis energy source is obtained from a laser source. The photolysis can be performed from outside the container (for example shining the light beam through the container walls in the case of a transparent container) or inside the container (for example with a collinear annular beam directed from the open end to the second end of the container). The source would be a tunable (selective) light source consisting for example of a tunable laser (using dye, or n-harmonic generation crystals) or a white light source coupled to a filter, for example a laser-driven-light source (such as LDLS EQ-99 from Energetiq Technology, Inc. of MA). In the case of photolysis, the filament or other heating source can be used to enhance the catalysis but is not required not for performing the pyrolysis of the monomer gas.

As shown in Fig. 9, the photolysis energy source 250 can be positioned at least partially within the chamber 226. Alternatively or additionally, the photolysis source 251 can be positioned externally of the chamber 226.

In some non-limiting embodiments, the gas inlet duct 236 can further comprise a catalyst which activates products from the photolysis reaction to form the reactive gas comprising at least one reactive moiety. The catalyst can comprise a transition metal catalytic surface positioned within the chamber, as discussed above, and/or a transition metal surface that may be incorporated into the gas inlet duct 36.

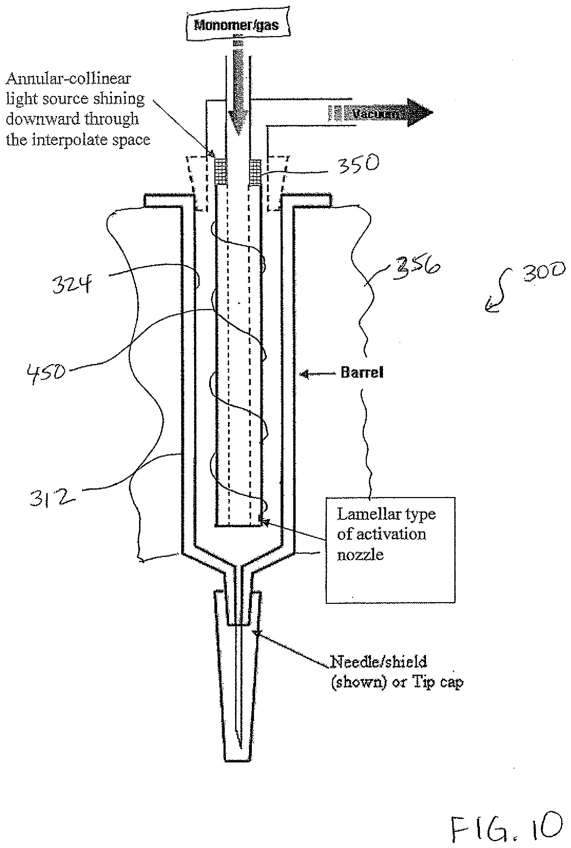

Referring now to Fig. 10, in some non-limiting embodiments, the system and apparatus can comprise both a photolysis energy source 350 and pyrolyzing surface 450, such as are described above. The pyrolyzing surface can pyrolyze at least a portion of the monomer gas or photolysis product(s) to form a reactive gas comprising at least one reactive moiety. The system can further comprise a temperature controller 356 for maintaining the interior wall surface 324 of the container 312 at a temperature which is less than the temperature of the pyrolyzing surface 350 and/or the photolysis energy source 350 to facilitate deposition and polymerization of the reactive moiety on at least a portion of the interior wall surface 324 of the container 312.

In some non-limiting embodiments, the present disclosure provides a method for applying a film coating to an interior wall surface of a container, comprising: (a) providing a monomer gas supply source for supplying at least one monomer gas to a gas inlet duct; (b) positioning a container comprising an open end, a second end opposite the open end, and a wall extending therebetween, the wall having an exterior wall surface and an interior wall surface, the container having a chamber within an area defined by the interior wall surface between the open end and the second end of the container, such that the open end is connected to the monomer gas supply source by the gas inlet duct; (c) positioning the gas inlet duct at the open end of the container such that a portion of the gas inlet duct extends into a portion of the chamber for supplying at least one monomer gas received from the monomer gas supply source into the chamber; (d) photolyzing at least a portion of the monomer gas supplied to the chamber of the container to form a reactive gas comprising at least one reactive moiety; and (e) removing excess reactive gas from the chamber through an outlet duct positioned at the open end of the container or the second end of the container. In some non-limiting embodiments, the method further comprises exposing the reactive gas to a catalyst to activate product from the photolysis reaction to form the reactive gas comprising at least one reactive moiety. In some non-limiting embodiments, the method further comprises pyrolyzing at least a portion of the monomer gas to form a reactive gas comprising at least one reactive moiety.

The pyrolyzing/photolyzing step can be taken prior or simultaneously with the activation or enhancement of the reactive specie(s) for the polymerization step, wherein in pyrolyzing CVD the pyrolyzing step is done simultaneously. This allows the use of better catalytic materials without imposing thermal constraints as well as facilitating coating deposition far from the pyrolysis/photolysis location.

In some non-limiting embodiments, the present disclosure provides a medical article, such as a container as described above, comprising a chamber having an interior wall surface and at least one coating layer thereon, wherein the coating layer has a first region deposited from a first reactive gas having a chemical functionality that is reactive with a first surface functionality present in a first domain of the interior wall surface prior to coating and a second region deposited from a second reactive gas having a chemical functionality that is reactive with a second surface functionality present in a second domain of the interior wall surface prior to coating. Such chemical functionalities can include carbon-carbon unsaturation, nitrile, imido, amido or halo functionality, for example.

Referring now to Fig. 7, the interior wall surface 24 of the container 12 can comprise regions or domains 74 of different chemical functionality or properties, for example a first domain 76 can have carbon to carbon double bond (unsaturated) chemical functionality and a second domain 78 can have ester chemical functionality. Reactive gases having different reactive functionalities can be selectively deposited onto these first and second domains 76, 78 (shown as corresponding first and second regions 80, 82) deposited from reactive gases having respective functionalities that are reactive with the surface functionality present in each domain 76, 78. For example, if the first domain 76 has C=C chemical functionality, a reactive gas such as dicyclopentene having diene chemical functionality can be deposited to react with the C=C chemical functionality of the first domain 76. Similarly, if the second domain 78 has ketone chemical functionality, a reactive gas such as oxygen having can be concurrently or sequentially deposited to react with the ketone chemical functionality of the second domain 78. Thus, the final coating can have regions 80, 82 of different chemical identities having different physical properties, for example hydrophilic and hydrophobic regions.

For example, the interior wall surface 24 of the container 12 can be formed from a mixture of cyclic polyolefin and SEBS, providing domains of SEBS rich in unsaturated bonds. The unsaturated bonds are more likely to react with the difluorocarbene radical than saturated carbon domains. Thus the regions with a heavier concentration of PTFE can provide regions of greater lubricity, and other regions can be tailored using other reactive gases to provide other physical characteristics, such as hydrophilicity or hydrophobicity.

The coating layer is formed by exposing the interior wall surface to the first and second reactive gases, either sequentially or simultaneously, and exposing the coated interior wall surface to an energy source to facilitate formation of the coating layer. In some non-limiting embodiments, the coating layer is applied by chemical vapor deposition, such as plasma CVD or hot filament CVD. In other non-limiting embodiments, the coating layer is exposed to oxidative treatment to facilitate formation of the coating layer, or a combination thereof.

Thus, the present disclosure provides a method for applying a film coating to an interior wall surface of a container, comprising: (a) providing a container comprising an interior wall surface, the interior wall surface comprising a first domain having a first chemical functionality and a second domain having a second, different chemical functionality; (b) exposing the interior wall surface to a first reactive gas having a functionality reactive with the first chemical functionality of the first domain and a second reactive gas having a functionality reactive with the second chemical functionality of the second domain, either sequentially or simultaneously; and (c) exposing the coated interior wall surface to an energy source to facilitate formation of the coating layer.

The coated containers prepared according to the methods of the present disclosure can be used in conjunction with other components of a medical article, such as a sealing member. The sealing member can be formed from any elastomeric or plastic material. Three primary synthetic thermoset elastomers typically are used in medical applications: polyisoprene rubber, silicone rubber, and butyl rubber. Of the three rubbers, butyl rubber has been the most common choice for articles due to its high cleanness and permeation resistance which enables the rubber to protect oxygen- and water-sensitive drugs. Suitable butyl rubbers include copolymers of isobutylene (about 97-98%) and isoprene (about 2-3%). The butyl rubber can be halogenated with chlorine or bromine. Non-limiting examples of suitable rubber stoppers include those available from West Pharmaceuticals, American Gasket Rubber, Stelmi, and Helvoet Rubber & Plastic Technologies BV. Other useful elastomeric copolymers include, without limitation, thermoplastic elastomers, thermoplastic vulcanizates, styrene copolymers such as styrene-butadiene (SBR or SBS) copolymers, styrene-isoprene (SIS) block polymers or styrene-isoprene/butadiene (SIBS), in which the content of styrene in the styrene block copolymer ranges from about 10% to about 70%, and preferably from about 20% to about 50%. Non-limiting examples of suitable styrene-butadiene stoppers are available from Firestone Polymers, Dow, Reichhold, Kokoku Rubber Inc., and Chemix Ltd. Other suitable thermoplastic elastomers are available from GLS, Tecknor Apex, AES, Mitsubishi and Solvay Engineered Polymers, for example. The elastomer composition can include, without limitation, antioxidants and/or inorganic reinforcing agents to preserve the stability of the elastomer composition.

In some embodiments, the sealing member can be a stopper, O-ring, plunger tip, or piston, for example. Syringe plunger tips or pistons typically are made of a compressible, resilient material such as rubber, because of the rubber's ability to provide a seal between the plunger and interior housing of the syringe. Syringe plungers, like other equipment used in the care and treatment of patients, have to meet high performance standards, such as the ability to provide a tight seal between the plunger and the barrel of the syringe.

The stopper can be coated with a CVD coating as described above, or with an organopolysiloxane coating. The coated chamber and/or coated sealing member can be subjected to oxidative treatment, for example, plasma treatment. The surface lubricant can be conventional silicone oil (organopolysiloxane) of viscosity about 100 to 1,000,000; 100 to 60,000; or preferably about 1,000 to 12,500 cSt. The surface lubricating layer may be applied by any conventional method, such as spraying or dipping the stopper into a solution, about 4% by weight, of the surface lubricant in a solvent such as chloroform, dichloromethane or preferably a chlorofluorocarbon, such as FREON™ TF. The surface lubricant may optionally be lightly crosslinked by oxidative treatment and/or radiation.

In some embodiments, the coated articles are subjected to a sterilization treatment. Commonly used sterilization techniques used for medical devices include autoclaving, ethylene oxide (EtO) or gamma irradiation, as well as more recently introduced systems that involve low-temperature gas plasma and vapor phase sterilants.

The present invention has been described with reference to specific details of particular embodiments thereof.

## Claims

1. A system (10) comprising:
(a) a container (12), of which at least a portion of an interior wall surface (24) is to be coated, comprising an open end (16), a second end (18) opposite the open end (16), and a wall (20) extending there between, the wall (20) having an exterior wall surface (22) and an interior wall surface (24), the container (12) having a chamber (26) within an area defined by the interior wall surface (24) between the open end (16) and the second end (18) of the container, wherein the chamber (26) is sealed from an exterior of the container (12) and the container (12) acts as its own vacuum chamber;
(b) a monomer gas supply source (32) containing at least one monomer gas (34);
(c) a gas inlet duct (36) positioned at the open end (16) of the container (12) and having a portion (38) extending into a portion (40) of the chamber (26) for supplying at least one monomer gas (36) received from the monomer gas supply source (32) into the chamber (26);
(d) a pyrolyzing surface (50) for pyrolyzing at least a portion of the monomer gas (34) supplied to the chamber (26) of the container (12) to form a reactive gas (52) comprising at least one reactive moiety from the monomer gas;
(e) a temperature controller (56) proximate to the exterior wall surface (22) of the container (12) to promote cooling of the exterior wall surface (22) for maintaining the interior wall surface (24) of the container (12) at a desired temperature to facilitate deposition and polymerization of the reactive moiety on at least a portion of the interior wall surface (24) of the container (12); and
(f) an outlet duct (68) positioned at the open end (16) of the container (12) or the second end (18) of the container (12) for removing excess reactive gas (52) from the chamber (26),
wherein the monomer gas comprises at least one monomer selected from the group consisting of organosilicon monomers, halocarbon monomers, azurine monomers, thiirane monomers, unsaturated olefinic monomers and mixtures thereof.

2. The system (10) according to claim 1, wherein the temperature controller (56) is a metal plate (58) having at least one aperture (60) therethrough for receiving the container (12) therein such that the exterior wall surface (22) of the container (12) is proximate to or in facing engagement with the aperture (60) of the metal plate (58).

3. The system (10) according to claim 1, wherein the container (12) is selected from the group consisting of syringes, drug cartridges, needleless injectors, liquid dispensing devices, liquid metering devices, sample collection tubes, catheters, vials and tubing.

4. The system (10) according to claim 3, wherein the container (12) comprises a syringe barrel.

5. The system (10) according to claim 4, wherein the container (12) comprises a syringe barrel having an open end (16), a second, opposite end (18) having a needle (28) or cannula attachable thereto, and optionally a shield or tip cap (30) covering at least a portion of the exterior of the attachable needle (28) or cannula.

6. The system (10) according to claim 1, wherein the halocarbon monomer is selected from the group consisting of hexafluoropropylene oxide, tetrafluoroethylene, hexafluorocyclopropane, octafluorocyclobutane, perfluorooctanesulfonyl fluoride, octafluoropropane, trifluoromethane, difluoromethane, difluorodichloromethane, difluorodibromomethane,
difluorobromomethane, difluorochloromethane, trifluorochloromethane, tetrafluorocyclopropane, tetrachlorodifluorocyclopropane, trichlorotrifluoroethane, dichlorotetrafluorocyclopropane and mixtures thereof.

7. The system (10) according to claim 1, wherein the gas inlet duct (36) has a plurality of apertures in a sidewall of the tube.

8. The system (10) according to claim 1, wherein the gas inlet duct (36) is prepared from a porous material.

9. The system (10) according to claim 7, wherein the gas inlet duct (36) further comprises at least one plate extending radially from an exterior sidewall of the tube proximate an aperture in the sidewall of the tube.

10. The system (10) according to claim 1, wherein the pyrolyzing surface (50) comprises a hot filament wire.

## Patentansprüche

1. System (10), umfassend:
(a) einen Behälter (12), wobei mindestens ein Abschnitt einer Innenwandfläche (24) davon zu beschichten ist, umfassend ein offenes Ende (16), ein zweites Ende (18) gegenüber dem offenen Ende (16) und eine Wand (20), die sich dazwischen erstreckt, wobei die Wand (20) eine Außenwandfläche (22) und eine Innenwandfläche (24) aufweist, wobei der Behälter (12) eine Kammer (26) innerhalb eines Bereichs aufweist, der durch die Innenwandfläche (24) zwischen dem offenen Ende (16) und dem zweiten Ende (18) des Behälters definiert ist, wobei die Kammer (26) von einer Außenseite des Behälters (12) abgedichtet ist und der Behälter (12) als seine eigene Vakuumkammer wirkt;
(b) eine Monomer-Gaszufuhrquelle (32), umfassend mindestens ein Monomergas (34);
(c) einen Gaseinlasskanal (36), der am offenen Ende (16) des Behälters (12) angeordnet ist und einen Abschnitt (38) aufweist, der sich in einen Abschnitt (40) der Kammer (26) erstreckt, um mindestens ein Monomergas (36) zuzuführen, das von der Monomer-Gaszufuhrquelle (32) in der Kammer (26) aufgenommen wird;
(d) eine Pyrolysierfläche (50) zum Pyrolysieren mindestens eines Teils des Monomergases (34), das der Kammer (26) des Behälters (12) zugeführt wird, um ein reaktives Gas (52) zu bilden, das mindestens eine reaktive Einheit aus dem Monomergas umfasst;
(e) eine Temperatursteuerung (56) in der Nähe der Außenwandfläche (22) des Behälters (12) zum Fördern der Kühlung der Außenwandfläche (22) zum Beibehalten der Innenwandfläche (24) des Behälters (12) auf einer gewünschten Temperatur, um die Ablagerung und Polymerisation der reaktiven Einheit an mindestens einem Abschnitt der Innenwandfläche (24) des Behälters (12) zu erleichtern; und
(f) einen Auslasskanal (68), der am offenen Ende (16) des Behälters (12) oder am zweiten Ende (18) des Behälters (12) angeordnet ist, um überschüssiges reaktives Gas (52) aus der Kammer (26) zu entfernen,
wobei das Monomergas mindestens ein Monomer umfasst, das ausgewählt ist aus der Gruppe, bestehend aus Organosilicium-Monomeren, Halogenkohlenstoff-Monomeren, Azurinmonomeren, Thiiranmonomeren, ungesättigten olefinischen Monomeren und Mischungen davon.

2. System (10) nach Anspruch 1, wobei die Temperatursteuerung (56) eine Metallplatte (58) ist, die mindestens eine Öffnung (60) dadurch aufweist, um den Behälter (12) darin aufzunehmen, so dass die Außenwandfläche (22) des Behälters (12) in der Nähe der Öffnung (60) der Metallplatte (58) oder in gegenüberliegender Anlage damit ist.

3. System (10) nach Anspruch 1, wobei der Behälter (12) ausgewählt ist aus der Gruppe, bestehend aus Spritzen, Medikamentenkartuschen, nadellosen Injektoren, Flüssigkeitsabgabevorrichtungen, Flüssigkeitsdosiervorrichtungen, Probensammelröhrchen, Kathetern, Phiolen und Schläuchen.

4. System (10) nach Anspruch 3, wobei der Behälter (12) einen Spritzenzylinder umfasst.

5. System (10) nach Anspruch 4, wobei der Behälter (12) einen Spritzenzylinder umfasst, der ein offenes Ende (16), ein zweites, gegenüberliegendes Ende (18), das eine daran befestigbare Nadel (28) oder Kanüle aufweist, und gegebenenfalls eine Schutzkappe oder Spitzenkappe (30) aufweist, die mindestens einen Abschnitt der Außenseite der befestigbaren Nadel (28) oder Kanüle bedeckt.

6. System (10) nach Anspruch 1, wobei das Halogenkohlenstoff-Monomer ausgewählt ist aus der Gruppe, bestehend aus Hexafluorpropylenoxid, Tetrafluorethylen, Hexafluorcyclopropan, Octafluorcyclobutan, Perfluoroctansulfonylfluorid, Octafluorpropan, Trifluormethan, Difluormethan, Difluordichlormethan, Difluorodibrommethan, Difluorbrommethan, Difluorchlormethan, Trifluorchlormethan, Tetrafluorcyclopropan, Tetrachlordifluorcyclopropan, Trichlortrifluorethan, Dichlortetrafluorcyclopropan und Mischungen davon.

7. System (10) nach Anspruch 1, wobei der Gaseinlasskanal (36) eine Vielzahl von Öffnungen in einer Seitenwand der Rohrleitung aufweist.

8. System (10) nach Anspruch 1, wobei der Gaseinlasskanal (36) aus porösem Material hergestellt ist.

9. System (10) nach Anspruch 7, wobei der Gaseinlasskanal (36) weiterhin mindestens eine Platte umfasst, die sich radial von einer äußeren Seitenwand der Rohrleitung nahe einer Öffnung in der Seitenwand der Rohrleitung erstreckt.

10. System (10) nach Anspruch 1, wobei die Pyrolisierfläche (50) einen Heizfaden umfasst.

## Revendications

1. Système (10) comprenant :
(a) un récipient (12), dont au moins une partie d'une surface de paroi intérieure (24) doit être revêtue, comprenant une extrémité ouverte (16), une deuxième extrémité (18) opposée à l'extrémité ouverte (16), et une paroi (20) s'étendant entre celles-ci, la paroi (20) ayant une surface de paroi extérieure (22) et une surface de paroi intérieure (24), le récipient (12) ayant une chambre (26) à l'intérieur d'une zone définie par la surface de paroi intérieure (24) entre l'extrémité ouverte (16) et la deuxième extrémité (18) du récipient, où la chambre (26) est étanche par rapport à l'extérieur du récipient (12) et le récipient (12) agit comme sa propre chambre à vide ;
(b) une source d'alimentation en gaz monomère (32) contenant au moins un gaz monomère (34) ;
(c) un conduit d'entrée de gaz (36) positionné à l'extrémité ouverte (16) du récipient (12) et ayant une partie (38) s'étendant à l'intérieur d'une partie (40) de la chambre (26) pour fournir au moins un gaz monomère (36) reçu à partir de la source d'alimentation en gaz monomère (32) dans la chambre (26) ;
(d) une surface de pyrolyse (50) pour pyrolyser au moins une partie du gaz monomère (34) fourni à la chambre (26) du récipient (12) pour former un gaz réactif (52) comprenant au moins un fragment réactif provenant du gaz monomère ;
(e) un dispositif de commande de température (56) à proximité de la surface de paroi extérieure (22) du récipient (12) pour favoriser le refroidissement de la surface de paroi extérieure (22) afin de maintenir la surface de paroi intérieure (24) du récipient (12) à une température souhaitée pour faciliter la déposition et la polymérisation du fragment réactif sur au moins une partie de la surface de paroi intérieure (24) du récipient (12) ; et
(f) un conduit de sortie (68) positionné à l'extrémité ouverte (16) du récipient (12) ou à la deuxième extrémité (18) du récipient (12) pour évacuer l'excès de gaz réactif (52) de la chambre (26),
dans lequel le gaz monomère comprend au moins un monomère choisi dans le groupe constitué de monomères d'organo-silicium, de monomères d'hydrocarbure halogéné, de monomères d'azurine, de monomères de thiirane, de monomères oléfiniques insaturés et de leurs mélanges.

2. Système (10) selon la revendication 1, dans lequel le dispositif de commande de température (56) est une plaque métallique (58) traversée par au moins une ouverture (60) pour recevoir le récipient (12) dans celle-ci de sorte que la surface de paroi extérieure (22) du récipient (12) soit à proximité de ou disposée face à l'ouverture (60) de la plaque métallique (58).

3. Système (10) selon la revendication 1, dans lequel le récipient (12) est choisi dans le groupe constitué de seringues, de cartouches de médicament, d'injecteurs sans aiguille, de dispositifs de distribution de liquide, de dispositifs de dosage de liquide, de tubes de prélèvement d'échantillons, de cathéters, de flacons et de tubulure.

4. Système (10) selon la revendication 3, dans lequel le récipient (12) comprend un cylindre de seringue.

5. Système (10) selon la revendication 4, dans lequel le récipient (12) comprend un cylindre de seringue ayant une extrémité ouverte (16), une deuxième extrémité opposée (18) ayant une aiguille (28) ou une canule attachable, et éventuellement une protection ou un capuchon d'extrémité (30) couvrant au moins une partie de l'extérieur de l'aiguille (28) ou de la canule attachable.

6. Système (10) selon la revendication 1, dans lequel le monomère d'hydrocarbure halogéné est choisi dans le groupe constitué de l'oxyde d'hexafluoropropylène, du tétrafluoroéthylène, de l'hexafluorocyclopropane, de l'octafluorocyclobutane, du fluorure de perfluorooctanesulfonyle, de l'octafluoropropane, du trifluorométhane, du difluorométhane, du difluorodichlorométhane, du difluorodibromométhane, du difluorobromométhane, du difluorochlorométhane, du trifluorochlorométhane, du tétrafluorocyclopropane, du tétrachlorodifluorocyclopropane, du trichlorotrifluoroéthane, du dichlorotétrafluorocyclopropane et de leurs mélanges.

7. Système (10) selon la revendication 1, dans lequel le conduit d'entrée de gaz (36) a une pluralité d'ouvertures dans une paroi latérale du tube.

8. Système (10) selon la revendication 1, dans lequel le conduit d'entrée de gaz (36) est préparé d'un matériau poreux.

9. Système (10) selon la revendication 7, dans lequel le conduit d'entrée de gaz (36) comprend en outre au moins une plaque s'étendant radialement à partir d'une paroi latérale extérieure du tube à proximité d'une ouverture dans la paroi latérale du tube.

10. Système (10) selon la revendication 1, dans lequel la surface de pyrolyse (50) comprend un fil de filament chaud.
